# EUROPÄISCHE PATENTSCHRIFT

(11) **EP 4 260 897 B1**
(45) Veröffentlichungstag und Bekanntmachung des Hinweises auf die Patenterteilung: **03.06.2026**
(21) Anmeldenummer: 22168332.9
(22) Anmeldetag: 14.04.2022
(51) Int. Cl.: A61M 37/00

(54) **ANTRIEBSVORRICHTUNG FÜR EIN HANDGERÄT ZUM LOKALEN AUFSTECHEN EINER HAUT UND HANDGERÄT**
DRIVING DEVICE FOR A HAND TOOL FOR THE LOCAL PIERCING OF A SKIN AND HAND-HELD DEVICE
DISPOSITIF D'ENTRAINEMENT POUR UN APPAREIL PORTATIF PERMETTANT D'INCISER LOCALEMENT UNE PEAU ET APPAREIL PORTATIF

(43) Veröffentlichungstag der Anmeldung: 18.10.2023
(73) Patentinhaber: MT.DERM GmbH, 12307 Berlin (DE)
(72) Erfinder: SCHERKOWSKI, Dirk, 12489 Berlin (DE)
(74) Vertreter: Bittner, Thomas L.

(56) Entgegenhaltungen:
- EP-A1- 2 149 388
- EP-A1- 2 954 925
- FR-A1- 2 972 933
- KR-A- 20180 116 824
- US-A1- 2021 353 924

## Beschreibung

Die Erfindung betrifft eine Antriebsvorrichtung für ein Handgerät zum lokalen Aufstechen einer menschlichen oder einer tierischen Haut sowie ein Handgerät.

### Hintergrund

EP 2 954 925 A1 und EP 2 149 388 A1 betreffen Handgeräte zum wiederholten Aufstechen einer menschlichen oder tierischen Haut.

KR 2018 0116824 A betrifft eine Nadeleinheit für eine Tätowiervorrichtung mit vorratsstandunabhängig quantitativ steuerbarer Tintenzufuhr.

US 2021/353924 A1 beschreibt eine kabellose Tätowiervorrichtung.

FR 2 972 933 A1 betrifft eine professionelle Tätowiervorrichtung.

Solche Handgeräte werden verwendet, um eine Haut lokal aufzustechen, insbesondere zum Einbringen eines Farbstoffs in die Haut für ein Tattoo oder permanentes Makeup, aber auch zum Applizieren medizinischer oder kosmetischer Wirkstoffe auf die Haut, die mittels lokalen Aufstechens der Haut eingebracht werden. Die Handgeräte verfügen üblicherweise über eine Antriebsvorrichtung, die mit einem Gehäuse gebildet ist. In dem Gehäuse ist eine Antriebseinrichtung angeordnet, beispielsweise ein elektrischer Motor, mit dem eine drehende Antriebsbewegung bereitgestellt wird. Mit Hilfe eines Wandlungsmechanismus wird die drehende Antriebsbewegung in eine repetierende Vor- und Zurückbewegung gewandelt, die ihrerseits auf eine Hautstecheinrichtung übertragen wird, um ein oder mehrere Stechnadeln der Hautstecheinrichtung zum lokalen Aufstechen der Haut vor- und zurückzubewegen. Die Hautstecheinrichtung ist in einem Hautstechmodul aufgenommen, welches lösbar oder nicht lösbar mit dem Gehäuse der Antriebsvorrichtung verbunden ist, derart, dass die mittels der Antriebseinrichtung bereitgestellte Antriebskraft zum Vor- und Zurückbewegen der einen oder der mehreren Nadeln der Hautstecheinrichtung hierauf übertragen werden kann. Üblicherweise werden Stechnadelspitzen der einen oder der mehreren Stechnadeln beim Vor- und Zurückbewegen der Stechnadeln durch eine vordere Gehäuseöffnung des Hautstechmoduls aus- und eingefahren.

Bei der Verwendung des Handgeräts für unterschiedliche Anwendungen besteht häufig der Wunsch, das Ausmaß oder die Länge der Vor- und Zurückbewegung der Stechnadeln einstellen zu können, beispielsweise zum Verändern einer Stechtiefe in die Haut. Hierzu ist es bekannt, eine Hublänge zwischen einem vorderen und einem hinteren Endpunkt der Vor- und Zurückbewegung der Stechnadeln einzustellen.

### Zusammenfassung

Aufgabe der Erfindung ist es, eine verbesserte Antriebsvorrichtung für ein Handgerät zum lokalen Aufstechen einer Haut sowie ein verbessertes Handgerät anzugeben, mit denen eine sichere und flexibel Einstellung einer Hublänge der als Antriebsbewegung bereitgestellten repetierenden Vor- und Zurückbewegung ermöglicht wird.

Zur Lösung ist eine Antriebseinrichtung für ein Handgerät zum lokalen Aufstechen einer Haut nach dem unabhängigen Anspruch 1 geschaffen. Weiterhin ist ein Handgerät zum lokalen Aufstechen einer Haut nach dem nebengeordneten Anspruch 14 geschaffen. Ausgestaltungen sind Gegenstand von abhängigen Unteransprüchen.

Nach einem Aspekt ist eine Antriebsvorrichtung für ein Handgerät zum lokalen Aufstechen einer Haut geschaffen, welche Folgendes aufweist: ein Gehäuse, welches mit einem Hautstechmodul verbindbar ist; eine Antriebseinrichtung, die in dem Gehäuse angeordnet und eingerichtet ist, eine Antriebskraft mittels einer drehenden Antriebsbewegung einer Antriebswelle bereitzustellen, deren Axialrichtung quer zu einer Arbeitsrichtung verläuft; ein Verbindungsbauteil, welches eingerichtet ist, getrieben von der Antriebskraft, entlang der Arbeitsrichtung eine Vor- und Zurückbewegung mit einer Hublänge auszuführen; eine Verbindungsstelle, die an dem Verbindungsbauteil gebildet und zum Verbinden mit einer Hautstecheinrichtung des Hautstechmoduls eingerichtet ist; ein Wandlungsmechanismus, welcher, die Antriebskraft übertragend, zwischen der Antriebswelle und dem Verbindungsbauteil in dem Gehäuse angeordnet ist und eine Schwenkhebelanordnung aufweist, wobei bei dem Wandlungsmechanismus die Schwenkhebelanordnung mit einem Schwenkhebel gebildet ist, mit dem die drehende Antriebsbewegung der Antriebswelle abgreifbar ist und der hierbei um einen Drehpunkt schwenkt, der in Bezug auf die Antriebswelle radial versetzt ist, und ein Pleuelbauteil auf einer Seite mit einem Abgriff an dem Schwenkhebel und gegenüberliegend mit dem Verbindungsbauteil verbunden ist; und eine Hubeinstelleinrichtung, mit der zum Einstellen der Hublänge der Vor- und Zurückbewegung entlang der Arbeitsrichtung der Abgriff an dem Schwenkhebel verlagerbar ist.

Nach einem weiteren Aspekt ist ein Handgerät zum lokalen Aufstechen einer Haut mit der Antriebsvorrichtung sowie einem Hautstechmodul geschaffen, welches mit der Antriebsvorrichtung verbunden ist.

Mit Hilfe der Antriebsvorrichtung wird eine Antriebskraft zur Abgabe über das Verbindungsbauteil bereitgestellt, derart, dass die Antriebskraft über das Verbindungsbauteil auf eine Hautstecheinrichtung eines Hautstechmoduls, welches mit der Antriebsvorrichtung verbindbar ist, eingeleitet werden kann, um ein oder mehrere Stechnadeln der Hautstecheinrichtung zum lokalen Aufstechen einer Haut vor- und zurückzubewegen. Die bereitgestellte Vor- und Zurückbewegung ist durch eine Hublänge charakterisiert, also einen Abstand zwischen vorderem und hinteren Endpunkt der Vor- und Zurückbewegung entlang der Arbeitsrichtung. Wird die bereitgestellte Antriebsbewegung entlang der Arbeitsrichtung unverändert auf die Hautstecheinrichtung übertragen, führt dies im Betrieb zu einer Vor- und Zurückbewegung der Hautstecheinrichtung mit einer Arbeitshublänge, welche der mittels der Antriebsvorrichtung bereitgestellten Hublänge entspricht. Der Arbeitshub wird von der Hautstecheinrichtung, insbesondere den hiervon umfassten Stechnadeln, im Betrieb ausgeführt. Mittels einer optionalen Hubumsetzung kann die Arbeitshublänge wahlweise kürzer oder länger als die Hublänge ausgebildet werden.

Die von der Antriebsvorrichtung bereitgestellte Hublänge ist mit Hilfe der Hubeinstelleinrichtung veränderbar. Hierzu ist an dem Schwenkhebel der Abgriff verlagerbar. So ist auf einfache und zuverlässige Art und Weise eine Einstellung der Hublänge, die mit Hilfe der Antriebsvorrichtung bereitgestellt wird, für unterschiedliche Anwendungen beim lokalen Aufstechen der Haut ermöglicht.

Die Verbindungsstelle kann für ein gelenkige Verbindung des Verbindungsbauteils mit der Hautstecheinrichtung eingerichtet sein. Beispielsweise kann an dem Verbindungsbauteil im Bereich der Verbindungsstelle ein Kugelkopfgelenkbauteil vorgesehen sein. Die Verbindungsstelle kann in einer Führung angeordnet sein, die sich in der Arbeitsrichtung erstreckt.

Die Antriebseinrichtung kann eine maschinelle Antriebseinrichtung sein, die mit einem elektrischen Motor ausgeführt ist, der zum Beispiel als ein Außenläufer-Motor ausgeführt sein kann.

Die Antriebsvorrichtung kann eine Messeinrichtung aufweisen, die der Hubeinstelleinrichtung zugeordnet und eingerichtet ist, unterschiedliche Verlagerungsstellungen des Abgriffs an dem Schwenkhebel zu erfassen. Mit Hilfe der Messeinrichtung werden Messwerte erfasst, die die unterschiedlichen Verlagerungsstellungen des Abgriffs an dem Schwenkhebel anzeigen. Beispielsweise können elektronische Messwerte erfasst werden, zum Beispiel unter Verwendung eines Potentiometers oder eines anderen Messwertgebers, dessen unterschiedliche Stellungen den verschiedenen Verlagerungsstellungen des Abgriffs entsprechen.

An dem Gehäuse der Antriebsvorrichtung kann eine Anzeigeeinrichtung vorgesehen sein, welche die verschiedenen Verlagerungsstellungen des Abgriffs anzeigt. Die Anzeige kann hierbei die mittels der Messeinrichtung erfassten Messwerte aufgreifen und anzeigen. Alternativ oder in Ergänzung zur Messeinrichtung kann eine mechanische Anzeigeeinrichtung vorgesehen sein, beispielsweise ein Drehbauteil mit Anzeigeelementen entlang der umlaufenden Oberfläche, mit dem die verschiedenen Verlagerungsstellungen des Abgriffs an dem Schwenkhebel an der Antriebsvorrichtung dem Benutzer angezeigt werden.

Die Hubeinstelleinrichtung kann mit einer manuellen Hubeinstelleinrichtung gebildet sein. Die manuelle Hubeinstelleinrichtung ermöglicht es dem Benutzer, den Abgriff an dem Schwenkhebel zum Einstellen der Hublänge mittels manueller Bedienung zu bewirken. Beispielsweise kann am Gehäuse der Antriebsvorrichtdung ein Einstellrad oder ein Schiebeelement vorgesehen sein.

Die Hubeinstelleinrichtung kann mit einer elektronischen Hubeinstelleinrichtung gebildet sein. In Verbindung mit der elektronischen Hubeinstellung kann die Verlagerung des Abgriffs an dem Schwenkhebel mit Hilfe von elektronischen Steuersignalen an die Hubeinstelleinrichtung bewirkt werden. Diese kann hierzu zum Beispiel einen Schrittmotor umfassen, mit dem die Stellung des Abgriffs an dem Schwenkhebel in verschiedene Positionen relativ zum Schwenkhebel verlagert werden kann.

Die elektronischen Hubeinstelleinrichtung kann mit einer Steuereinrichtung verbunden sein, derart, dass von der Steuereinrichtung bereitstellbare Hubeinstellsignale an die elektronische Hubeinstelleinrichtung übertragbar sind. Die Steuereinrichtung kann in oder an dem Gehäuse der Antriebsvorrichtung angeordnet sein. Alternativ kann die Steuereinrichtung getrennt von der Antriebsvorrichtung gebildet sein und mit der elektronischen Hubeinstelleinrichtung über eine kabelgebundene oder kabellose Verbindung Steuersignale austauschen. Die Steuereinrichtung kann eingerichtet sein, auf eine Benutzerauswahl die elektronische Hubeinstelleinrichtung anzusteuern, so dass eine vom Benutzer vorgewählte Hublänge eingestellt wird.

Die Hubeinstelleinrichtung kann derart eingerichtet sein, dass der Abgriff an dem Schwenkhebel verlagerbar ist, während die drehende Antriebsbewegung über die Antriebswelle bereitgestellt wird. Bei dieser Ausführungsform ist es ermöglicht, die Hublänge der Vor- und Zurückbewegung während des Betriebs der Antriebsvorrichtung zu verändern, also während die drehende Antriebsbewegung von der Antriebseinrichtung bereitgestellt wird. Dieses unterstützt eine schnelle und flexible Änderung der Hublänge während des Betriebs der Antriebsvorrichtung.

Die Hubeinstelleinrichtung kann eingerichtet sein, beim Verlagern des Abgriffs an dem Schwenkhebel einen Abstand des Abgriffs zum Drehpunkt des Schwenkhebels zu ändern.

Die Antriebsvorrichtung kann eine Abgriffeinrichtung zum Abgreifen der drehenden Antriebsbewegung der Antriebswelle aufweisen, wobei die Abgriffeinrichtung ein Drehbauteil, welches mit der Antriebswelle drehfest verbunden ist, und ein mit dem Drehbauteil in Verbindung stehendes erstes Gleitbauteil aufweist, welches in einer ersten Gleitführung an dem Schwenkhebel verlagerbar angeordnet ist. Das erste Gleitbauteil kann an einem Kurbelzapfen angeordnet sein, welcher an Drehbauteil (exzentrisch in Bezug auf die Antriebswelle) angeordnet ist, zum Beispiel aufrecht stehend auf einer Oberfläche des Drehbauteils, die ihrerseits quer zur Axialrichtung der Antriebswelle steht. Der Kurbelzapfen ist in radialer Richtung versetzt zur Antriebswelle angeordnet. Das Drehbauteil kann Teil der Antriebseinrichtung sein, insbesondere des elektrischen Motors.

Mit Hilfe der Abgriffeinrichtung wird die über die Antriebswelle bereitgestellte drehende Antriebsbewegung abgegriffen. Das Drehbauteil dreht sich beim Drehen der Antriebswelle mit dieser, wodurch der Kurbelzapfen umläuft. Mit Hilfe des mit dem Drehbauteil in Verbindung stehenden ersten Gleitbauteils (am Kurbelzapfen) in der ersten Gleitführung an dem Schwenkhebel wird der Schwenkhebel aufgrund der drehenden Antriebsbewegung der Antriebswelle (und des Drehbauteils sowie des exzentrischen Kurbelzapfens) in eine Schwenkbewegung um den Drehpunkt versetzt. Das erste Gleitbauteil kann in der ersten Gleitführung entlang einer geraden oder einer gekrümmten Bewegungs- oder Gleitbahn geführt werden.

Der Abgriff kann an dem Schwenkhebel mit einem zweiten Gleitbauteil gebildet sein, welches in einer zweiten Gleitführung an dem Schwenkhebel verlagerbar angeordnet ist. Bei dieser Ausführungsform ist im Zusammenhang mit dem Abgriff an dem Schwenkhebel das zweite Gleitbauteil in der zweiten Gleitführung angeordnet, die an dem Schwenkhebel ausgebildet ist, insbesondere als von der ersten Gleitführung getrennt ausgeführte weitere Gleitführung. In der zweiten Gleitführung kann das zweite Gleitbauteil entlang einer geraden oder einer gekrümmten Bewegungsbahn hin und her bewegt werden.

Eine Längsrichtung der ersten Gleitführung und eine Längsrichtung der zweiten Gleitführung können einen spitzen Winkel einschließen. Ist zumindest eine der Gleitführungen als eine gekrümmte Gleitführung ausgeführt, so entspricht die Längsrichtung der gekrümmten Gleitführung der geraden Verbindung zwischen den gegenüberliegenden Enden der Gleitführung.

Das Pleuelbauteil und das zweite Gleitbauteil können im Bereich des Abgriffs relativ zueinander um eine Schwenkachse schwenkbar sein. Das Pleuelbauteil und das zweite Gleitbauteil können zum Beispiel mittels einer Stiftlagerung miteinander verbunden sein. Die Schwenkachse kann im Wesentlichen parallel zur Axialrichtung der Antriebswelle verlaufen.

Die Hubeinstelleinrichtung kann ein Einstellbauteil aufweisen, bei dem ein der Hubeinstelleinrichtung zugeordnetes und mit dem Abgriff verbundenes drittes Gleitbauteil in einer dritten Gleitführung an dem Einstellbauteil verlagerbar angeordnet ist. Das Einstellbauteil ist als Bestandteil der Hubeinstelleinrichtung verlagerbar, um zum Einstellen der Hublänge den Abgriff am Schwenkhebel zu verlagern. Führt der Schwenkhebel im Betrieb eine Schwenkbewegung aus, bewegt sich das der Hubeinstelleinrichtung zugeordnete dritte Gleitbauteil in der dritten Gleitführung an dem Einstellbauteil hin und her. Die dritte Gleitführung kann für das dritte Gleitbauteil eine gerade oder eine gekrümmte Führungsbahn bereitstellen. Die Längsrichtung der dritten Gleitführung kann im Wesentlichen quer zur Antriebswelle und / oder zur Arbeitsrichtung verlaufen. Das Einstellbauteil selbst verbleibt nach dem Verlagern in eine gewünschte Stellung (in Verbindung mit einer vorgesehenen Stellung des Abgriffs am Schwenkhebel) ortsfest, wenn die Antriebseinrichtung betrieben wird, es sei denn, die Hublänge soll geändert werden, was mit Hilfe einer Verlagerung des Einstellbauteils ermöglicht ist, wahlweise auch im laufenden Betrieb.

Das Pleuelbauteil und das der Hubeinstelleinrichtung zugeordnete dritte Gleitbauteil können im Bereich des Abgriffs relativ zueinander um die Schwenkachse schwenkbar sein.

Der Wandlungsmechanismus zum Übertragen der Antriebsbewegung kann frei von einer gemeinsamen Drehachse zwischen zweitem und drittem Gleitbauteil gebildet sein. Es kann alternativ eine gelenkige Verbindung anderswo vorgesehen sein, oder sogar ganz entfallen. Stattdessen kann eine andere Form der Kopplung vorgesehen sein, beispielsweise ein variabel seitlich versetztes Schieberbauteil, ein elastisches Pleueldrahtbauteil oder dergleichen.

Die Arbeitsrichtung kann in Längsrichtung des Gehäuses ausgebildet sein.

Die vorangehend im Zusammenhang mit der Antriebsvorrichtdung erläuterten Ausgestaltungen können in Verbindung mit dem Handgerät zum lokalen Aufstechen der Haut entsprechend vorgesehen sein.

Bei dem Handgerät kann das Hautstechmodul, bei dem in einem Modulgehäuse die Hautstecheinrichtung mit der einen oder der mehreren Stechnadeln angeordnet ist, lösbar oder nicht lösbar verbunden sein. Im Fall einer lösbaren Montage des Hautstechmoduls an der Antriebsvorrichtung, insbesondere an deren Gehäuse, kann das Hautstechmodul als ein Einwegmodul ausgeführt sein.

Mit dem Gehäuse der Antriebsvorrichtung kann ein Handstücks des Handgeräts bereitgestellt sein, welches vom Benutzer mit den Fingern gegriffen wird, um das Handgerät zum lokalen Aufstechen einer Haut zu führen.

An dem Gehäuse der Antriebsvorrichtung kann eine Einstelleinrichtung für eine Nadelherausstand vorgesehen. Die Einstelleinrichtung für den Nadelherausstand kann vom Gehäuse lösbar sein, wahlweise gemeinsam mit oder getrennt von dem Hautstechmodul. Mit Hilfe der Einstelleinrichtung für eine Nadelherausstand ist einstellbar, in welchem Umfang die Nadelspitzen in der vorderen Stellung über eine vordere Gehäusekante des Hautstechmoduls über- oder herausstehen. Hierzu kann die Einstelleinrichtung für den Nadelherausstand (NHS) ein NHS-Einstellbauteil aufweisen, welches mittels Drehen um eine Drehachse in Längsrichtung des Gehäuses in Bezug auf die Nadelsitzen zu diesen hin oder von diesen weg bewegt werden kann, wodurch die relative Lage der vorderen Gehäusekante zu den Nadelspitzen verändert wird, um den Nadelüberstand oder -herausstand einzustellen.

Die maschinelle Antriebseinrichtung, beispielsweise der elektrische Motor, wird über eine kabelgebundene oder eine kabellose Verbindung mit elektrischer Energie versorgt, um im Betrieb die drehende Antriebsbewegung bereitzustellen.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische Darstellung eines Handgeräts zum lokalen Aufstechen einer Haut;
- Fig. 2: eine schematische perspektivische Darstellung des Handgeräts aus Fig. 1;
- Fig. 3: eine schematische perspektivische Darstellung des Handgeräts aus Fig. 1, wobei ein Hautstechmodul von einer Antriebsvorrichtung gelöst ist;
- Fig. 4: eine schematische Darstellung des Handgeräts aus Fig. 1, wobei ein Teil eines Gehäuses einer Antriebsvorrichtung weggelassen ist;
- Fig. 5: eine schematische perspektivische Darstellung des Handgeräts aus Fig. 1 teilweise im Schnitt und ohne Gehäuse;
- Fig. 6: eine schematische Darstellung eines Wandlungsmechanismus des Handgeräts nach Fig. 1;
- Fig. 7: eine weitere schematische Darstellung des Wandlungsmechanismus aus Fig. 6 mit einer ersten Einstellung einer Hubeinstelleinrichtung;
- Fig. 8: eine weitere schematische Darstellung des Wandlungsmechanismus aus Fig. 6 mit einer zweiten Einstellung einer Hubeinstelleinrichtung und
- Fig. 9: eine schematische perspektivische Darstellung von Elementen des Wandlungsmechanismus und einer Antriebseinrichtung;
- Fig. 10: eine schematische perspektivische Darstellung des Handgeräts zum lokalen Aufstechen einer Haut, wobei Hautstechmodul und Griffstück vom Handstück getrennt sind;
- Fig. 11: eine schematische perspektivische Darstellung des Handgeräts aus Fig. 10 teilweise im Schnitt;
- Fig. 12: eine schematische perspektivische Darstellung des Handgeräts aus Fig. 11, wobei das Griffstück mit dem Handstück verbunden ist;
- Fig. 13: eine schematische perspektivische Darstellung des Handgeräts aus Fig. 11, wobei Hautstechmodul und Griffstück miteinander verbunden sind,
- Fig. 14: eine schematische perspektivische Detaildarstellung mit einer ersten Einstellung für den Nadelherausstand und
- Fig. 15: eine schematische perspektivische Detaildarstellung mit einer zweiten Einstellung für den Nadelherausstand.

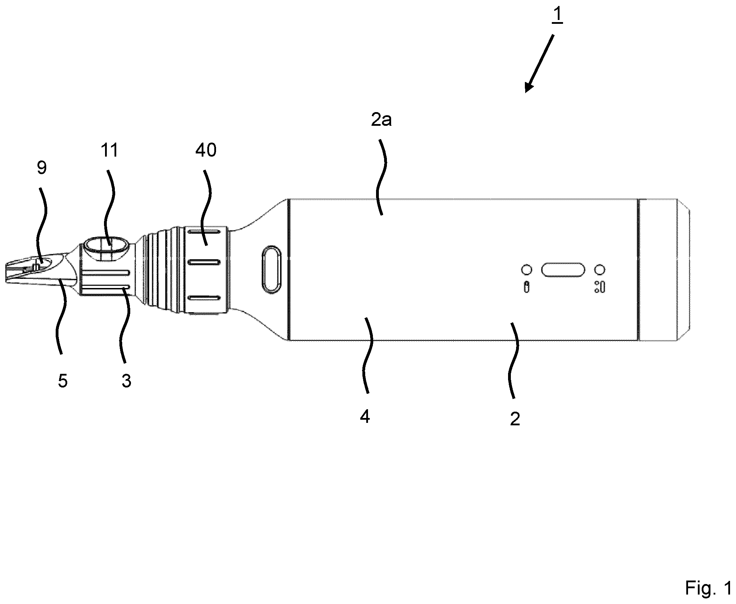

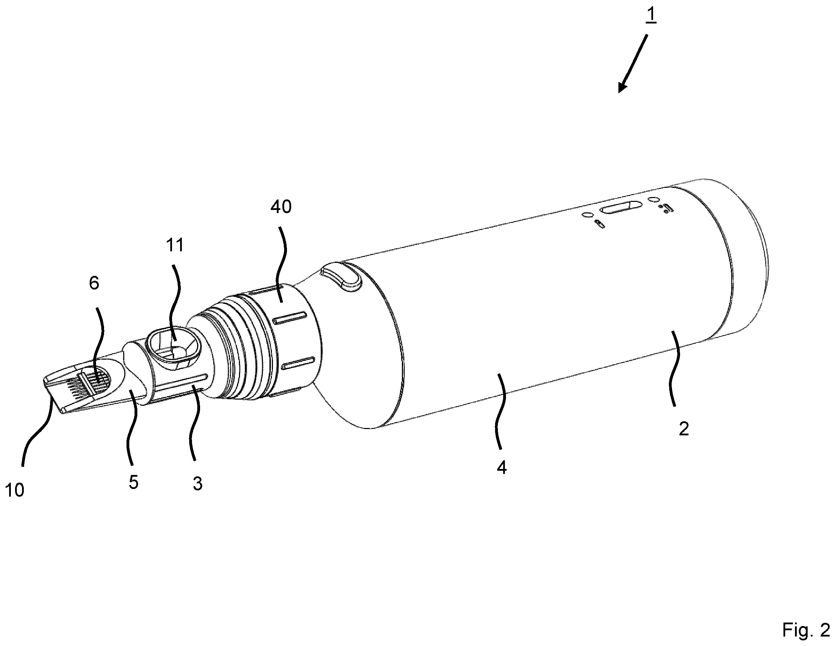

Fig. 1 bis 3 zeigen Darstellungen eines Handgeräts 1 zum lokalen Aufstechen einer menschlichen oder einer tierischen Haut. Das Handgerät 1 weist eine Antriebsvorrichtung 2 sowie ein hiermit verbundenes Hautstechmodul 3 auf, welches mit einem Gehäuse 4 der Antriebsvorrichtung 2 lösbar oder nicht lösbar verbunden ist. Ist das Hautstechmodul 3 mit der Antriebsvorrichtung 2 lösbar verbunden, kann es als Einwegmodul ausgeführt sein, so dass das Hautstechmodul 3 nach einer Verwendung entsorgt werden kann. Das Gehäuse 4 der Antriebsvorrichtung 2 wie auch ein Modulgehäuse 5 des Hautstechmoduls 3 können ein- oder mehrstückig ausgebildet sein.

Mit der Antriebsvorrichtung 2 kann ein Handstück 2a des Handgeräts 1 zum lokalen Aufstechen einer menschlichen oder einer tierischen Haut gebildet sein, welches vom Nutzer im Betrieb in die Hand oder die Finger genommen wird. Das Gehäuse 4 bildet dann ein Handstück-Gehäuse, welches zum Ausbilden des Handgeräts 1 mit dem Hautstechmodul 3 verbunden wird, insbesondere dessen Modulgehäuse 5.

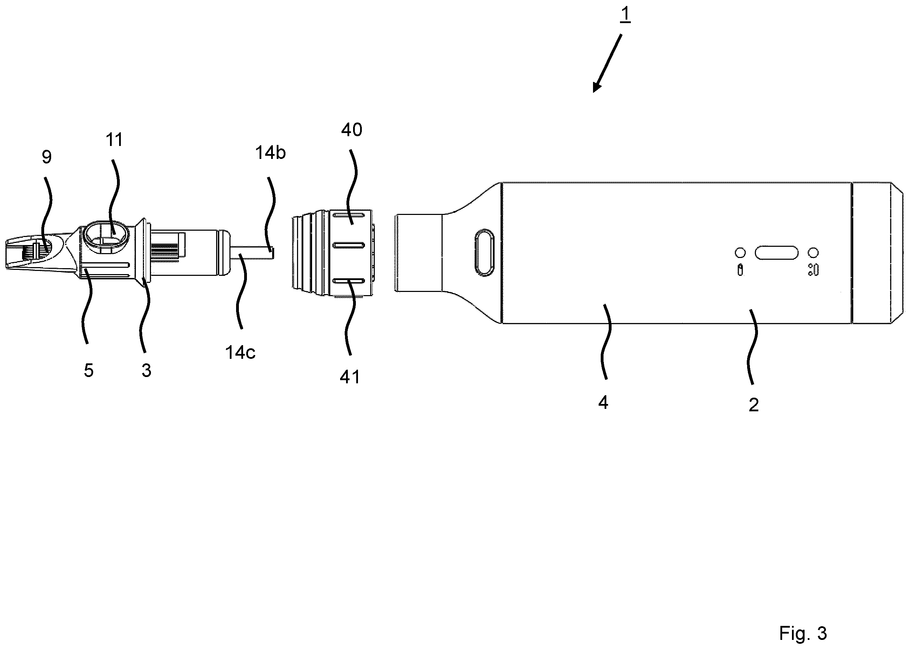

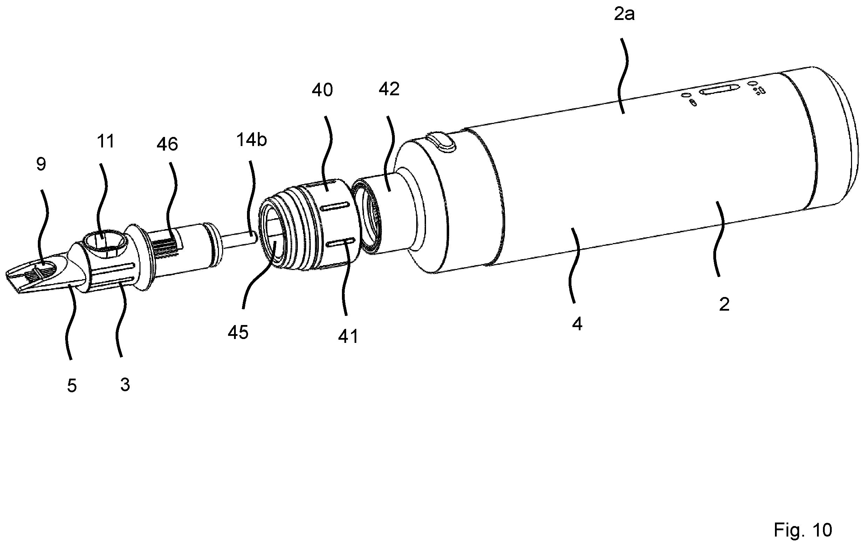

Das Hautstechmodul 3 ist gemäß Fig. 3 von der Antriebsvorrichtung 2 lösbar und somit austauschbar. Bei dem gezeigten Beispiel sind die Antriebsvorrichtung 2, insbesondere das Gehäuse 4, sowie das Hautstechmodul 3, insbesondere das Modulgehäuse 5, mit einem Griffstück 40 verbunden (vgl. weitere Erläuterungen unten zu Fig. 10 bis 15).

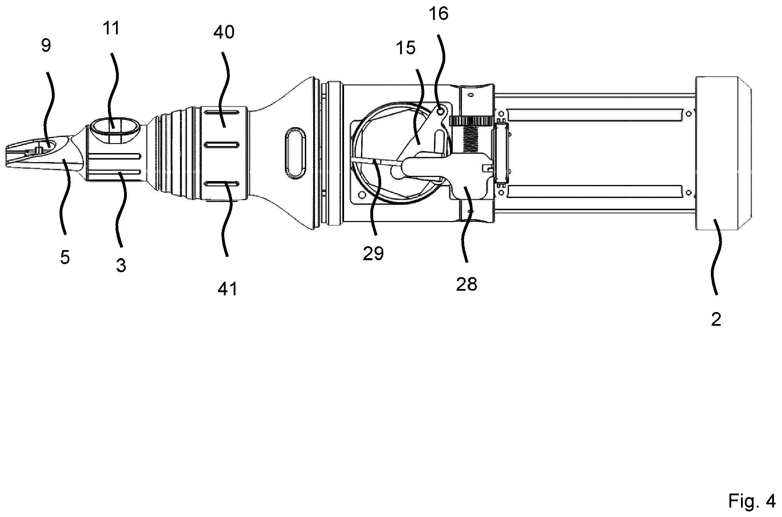

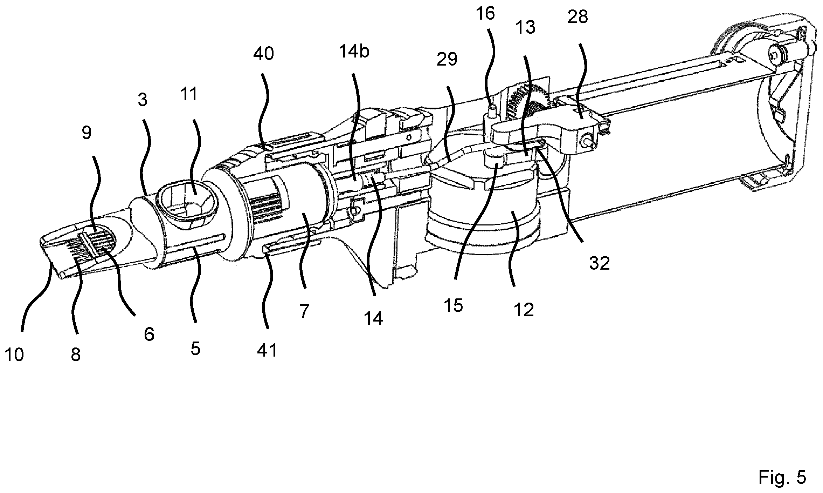

Fig. 4 und 5 zeigen schematische Darstellungen des Handgeräts 1, wobei das Handgerät 1 teilweise ohne das Gehäuse 4 der Antriebsvorrichtung 2 sowie teilweise im Schnitt dargestellt ist.

Zum lokalen Aufstechen einer Haut weist das Hautstechmodul 3 mehrere Stechnadeln 6 an einer Hautstecheinrichtung 7 auf, die im Modulgehäuse 5 des Hautstechmoduls 3 verlagerbar aufgenommen ist. Im Betrieb des Handgeräts 1 werden Nadelspitzen 8 der Stechnadel 6 durch eine vorderseitige Öffnung 9 des Modulgehäuses 5 aus- und eingefahren, derart, dass die Nadelspitzen 8 zumindest in einer ausgefahrenen Stellung (nicht dargestellt) gegenüber einer vorderen Gehäusekante 10 überstehen, so dass die zu behandelnde Haut lokal mittels der Nadelspitzen 8 aufgestochen werden kann. Ein Farbstoff und / oder ein Wirkstoff, die in Verbindung mit dem lokalen Aufstechen auf eine Haut appliziert werden sollen, können über eine Einfüllöffnung 11 am Modulgehäuse 5 zu den Stechnadeln 6 hin eingebracht werden. Alternativ kann am Gehäuse 4 oder am Modulgehäuse 5 zum Beispiel ein Vorratstank (nicht dargestellt) vorgesehen sein, welcher mit den Stechnadeln 6 in Fluidverbindung steht, so dass eine Flüssigkeit aus dem Vorratsbehälter zu den Stechnadeln 6 hin verbracht werden kann.

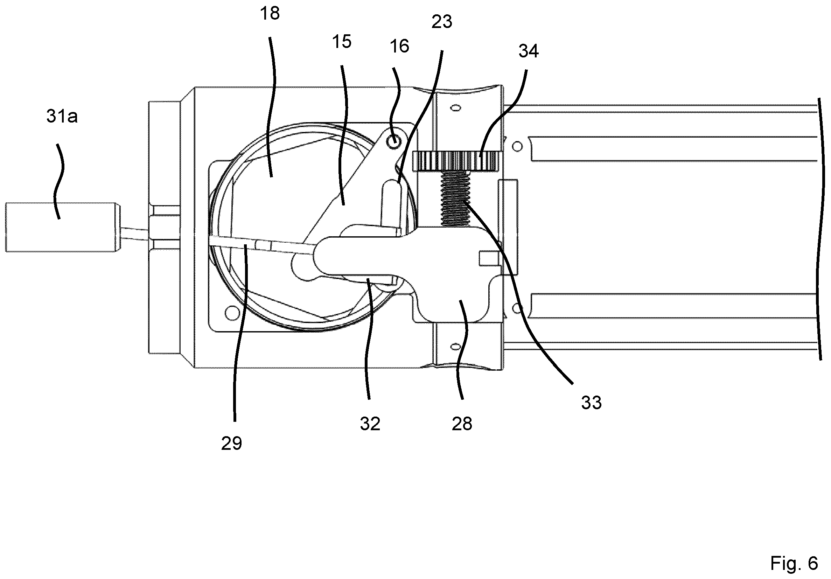

Um die Stechnadeln 6 im Betrieb des Handgeräts 1 in einer Linearbewegung vor- und zurückzubewegen, wird mit Hilfe einer Antriebseinrichtung 12, die beispielsweise mit einem elektrischen Motor gebildet ist, eine drehende Antriebsbewegung bereitgestellt. Mit Hilfe eines Wandlungsmechanismus 13, der nachfolgend insbesondere auch unter Bezugnahme auf die Fig. 6 bis 9 näher erläutert wird, wird eine Antriebskraft übertragen, die drehende Antriebsbewegung in eine Linearbewegung gewandelt und über ein Verbindungsbauteil 14 auf die Hautstecheinrichtung 7 mit den Stechnadeln 6 gekoppelt, so dass die Stechnadeln 6 im Betrieb aufgrund der eingekoppelten Antriebsbewegung eine erzwungene Vor- und Zurückbewegung entlang einer Arbeitsrichtung ausführen, die bei dem gezeigten Ausführungsbeispiel in Längsrichtung des Gehäuses 4 wie auch in Längsrichtung des Modulgehäuses 5 verläuft. An dem Verbindungsbauteil 14 ist eine (beispielweise handstückseitige) Verbindungsstelle 14a vorgesehen, die eingerichtet ist für eine (zum Beispiel lösbare) Verbindung zur Hautstecheinrichtung 7, sei es direkt oder indirekt (vermittelt über ein oder mehrere Zwischenbauteile).

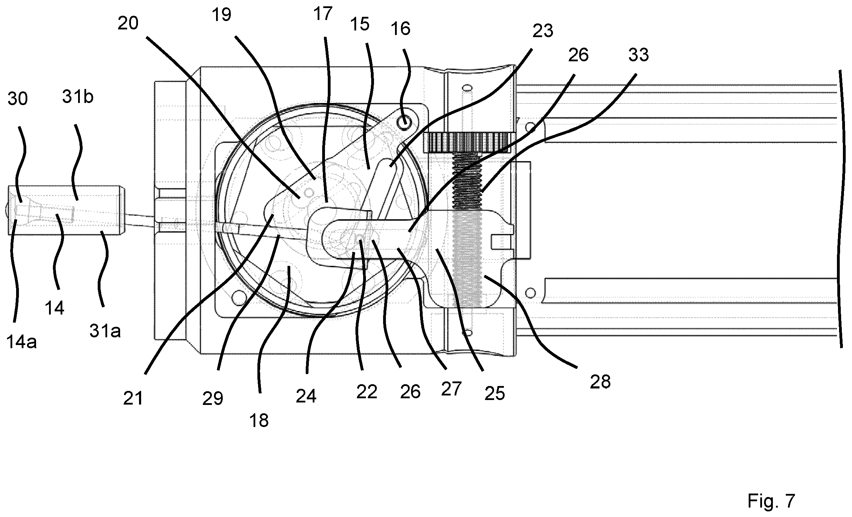

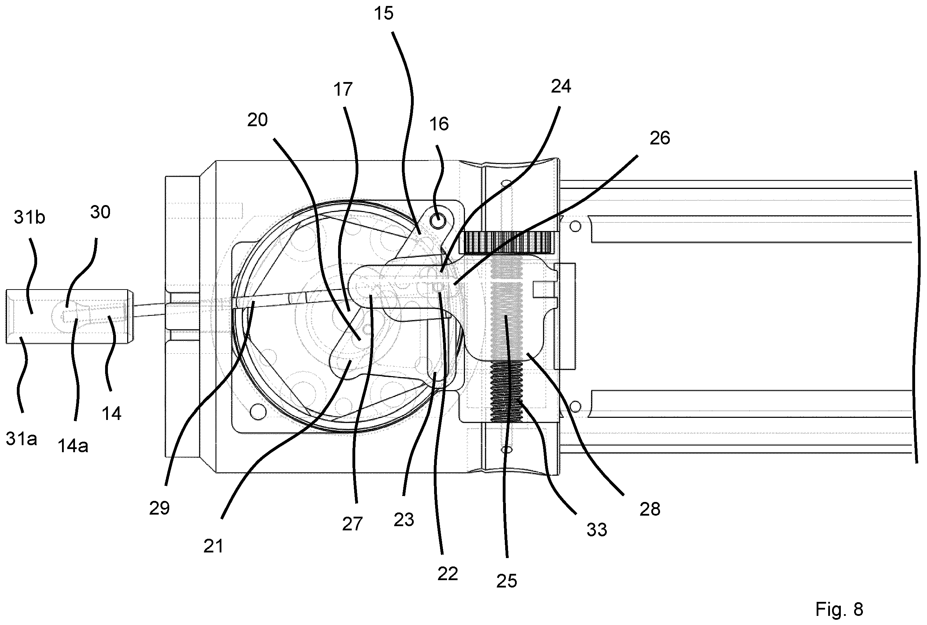

Bei dem Wandlungsmechanismus 13 ist ein Schwenkhebel 15 um einen Drehpunkt 16 schwenkbar gelagert, der in Bezug auf eine Antriebswelle 17 der Antriebseinrichtung 12 radial versetzt angeordnet ist (vgl. Fig. 7 und 8). Die Antriebswelle 17 erstreckt sich bei dem gezeigten Beispiel in einer Axialrichtung quer zur Arbeitsrichtung (also zur Längsrichtung des Gehäuses 4).

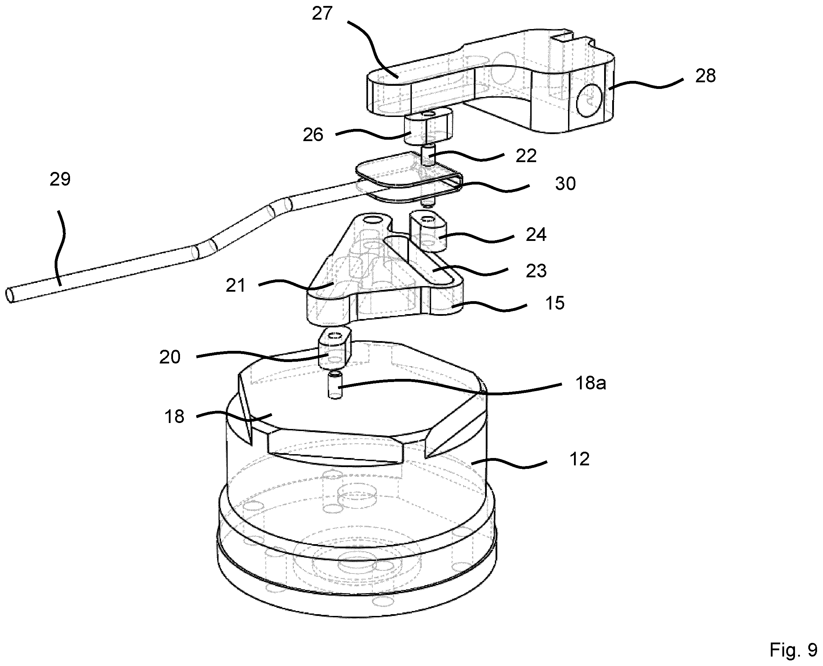

An der Antriebswelle 17 ist ein Drehbauteil 18 drehfest gelagert, so dass sich dieses beim Rotieren der Antriebswelle 17 mit dreht. Drehbauteil 18 ist Teil einer Abgriffeinrichtung 19, bei der das Drehbauteil 18 (zum Beispiel über einen Kurbelzapfen 18a; vgl. Fig. 9) mit einem ersten Gleitbauteil 20 in Verbindung steht, welches seinerseits beim Drehen der Antriebswelle 17 in einer ersten Gleitführung 21 an dem Schwenkhebel 15 bewegt wird. Das erste Gleitbauteil 20 ist an dem Kurbelzapfen 18a drehbar gelagert und bewegt sich in der ersten Gleitführung 21 hin und her, wenn das Drehbauteil 18 mit der Antriebswelle 17 gedreht wird. Auf diese Weise wird die Antriebsbewegung auf den Schwenkhebel 15 übertragen, der dann um den Drehpunkt 16 schwenkt.

An dem Schwenkhebel 15 ist ein Abgriff 22 angeordnet, welcher an dem Schwenkhebel 15 entlang einer zweiten Gleitführung 23 verlagerbar ist. In der zweiten Gleitführung 23 ist ein zweites Gleitbauteil 24 aufgenommen. Die erste und die zweite Gleitführung 21, 23 sind als getrennte Gleitführungen an dem Schwenkhebel 15 gebildet und schließen im gezeigten Beispiel einen spitzen Winkel ein.

Schwenkt der Schwenkhebel 15 aufgrund der Antriebsbewegung um den Drehpunkt 16, bewegt sich ein einer Hubeinstelleinrichtung 25 zugeordnetes drittes Gleitbauteil 26 in einer dritten Gleitführung 27 an einem Einstellbauteil 28 der Hubeinstelleinrichtung 25. Das zweite Gleitbauteil 24 und das der Hubeinstelleinrichtung 25 zugeordnete dritte Gleitbauteil 26 sind an einer gemeinsamen Stiftlagerung 28 relativ zueinander verschwenkbar gelagert. An der Stiftlagerung 28 ist weiterhin ein Pleuelbauteil 29 drehbar gelagert, welches so mit dem Abgriff 22 verbunden ist. Auf einer gegenüberliegenden Seite des Pleuelbauteils 29 ist dieses mit dem Verbindungsbauteil 14 verbunden, welches bei dem dargestellten Ausführungsbeispiel mit einem Kugelkopf 30 gebildet ist. Das Verbindungsbauteil 14 ist für eine gelenkige Verbindung mit der Hautstecheinrichtung 7 im Hautstechmodul 3 eingerichtet. Über das Pleuelbauteil 29 wird schließlich die Antriebsbewegung als lineare Vor- und Zurückbewegung auf die Hautstecheinrichtung 6 übertragen. Das Verbindungsbauteil 14 mit dem Kugelkopf 30 im Bereich der Verbindungsstelle 140 ist bei gezeigten Beispiel in einem Führungsbauteil 30a mit einer Führung 31b aufgenommen und bewegt sich bei der Vor- und Zurückbewegung entlang der Führung 31b (vgl. Fig. 7 und 8).

Die (handstückseitige) Verbindungsstelle 14b ist in dem gezeigten Beispiel an einer rückwärtigen Verlängerung 14c einer die Stechnadeln 6 tragend aufnehmenden Nadelaufnahme angeordnet und eingerichtet, zum Übertragen der Vor- und Zurückbewegung mit einer modulseitigen Verbindungsstelle 14b zusammenzuwirken.

Insbesondere gemäß Fig. 4 ist zwischen dem Schwenkhebel 15 und dem Einstellbauteil 28 im Bereich der Stiftlagerung ein Blechbauteil 32 angeordnet, welches mit dem Pleuelbauteil 29 eine Pleuelbaugruppe bilden kann.

Wie sich insbesondere aus den Fig. 6 bis 9 ergibt, ist das Einstellbauteil 28 der Hubeinstelleinrichtung 25 auf einer Gewindestange 33 angeordnet, so dass das Einstellbauteil 28 mittels Drehen der Gewindestange 33 mit Hilfe eines Einstellrads 34 quer zur Arbeitsrichtung sowie zur Längsrichtung des Gehäuses 4 verlagerbar ist. Auf diese Weise wird der Abgriff 22 am Schwenkhebel 15 verlagert, wobei insbesondere ein Abstand zum Drehpunkt 16 vergrößert oder verkleinert wird. Dieses führt dazu, dass die Hublänge der am Verbindungsbauteil 14 bereitgestellten Vor- und Zurückbewegung in Arbeitsrichtung vergrößert oder verkleinert wird. In den Fig. 6 und 7 sind unterschiedliche Stellungen für das Einstellbauteil 28 entlang der Gewindestange 33 gezeigt, die ihrerseits zu unterschiedlichen Stellungen oder Positionen des Abgriffs 22 an dem Schwenkhebel 15 führen, und damit zu unterschiedlicher Hublänge.

Mittels Wahl eines Winkels zwischen der ersten und der zweiten Gleitführung 21, 23 am Schwenkhebel 15 ergibt sich, dass in einer hinteren Betriebslage (siehe Fig. 8) die zweite Gleitführung 21 auf voller Länge genau quer zur Längsachse zu liegen kommt, was dazu führt, dass für alle Hubeinstellungen der hintere Umkehrpunkt weitestgehend konstant bleibt. Eine vollständige Konstanz kann ausgebildet werden, indem die zweite Gleitführung 23 kreisbogenförmig mit einem Mittelpunkt identisch dem des Kugelkopfes 30 am Verbindungsbauteil 14 gewählt wird.

Fig. 10 bis 15 zeigen weitere schematische perspektivische Darstellungen des Handgeräts 1.

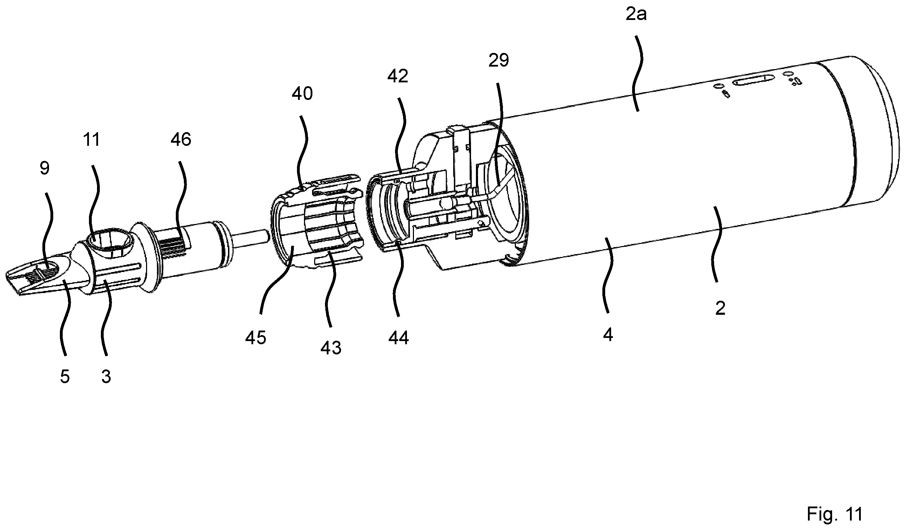

Gemäß Fig. 10 sind bei dem Handgerät 1 das Hautstechmodul 3, das Griffstück 40 mit Griffelementen 41 sowie die hier ein Handstück 2a bildende Antriebsvorrichtung 2 voneinander trennbar oder lösbar. Fig. 11 zeigt die getrennte Anordnung teilweise im Schnitt. Mit dem Gehäuse 4 ist ein Handstück-Gehäuse gebildet.

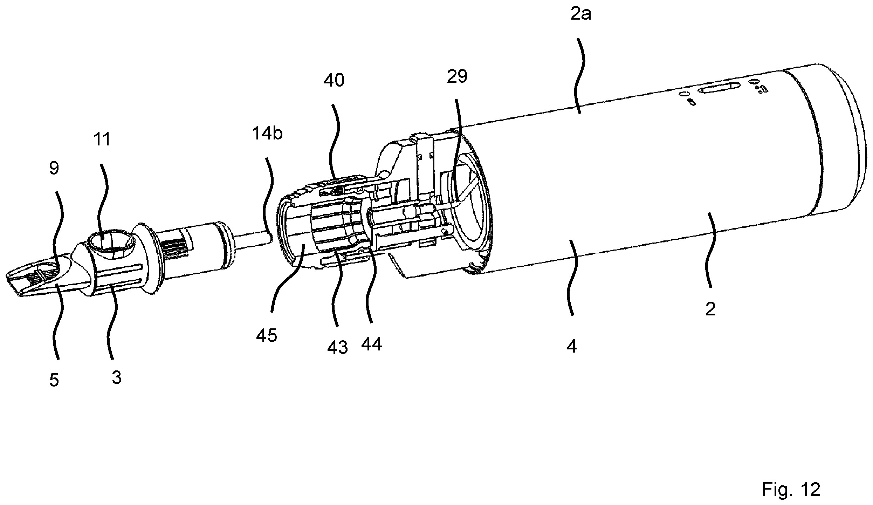

Um bei dem gezeigten Ausführungsbeispiel das Handstück 2a, also das Gehäuse 4 (Handstück-Gehäuse), das Griffstück 40 sowie das Hautstechmodul 3 miteinander funktional zu verbinden, wird zum (zumindest teilweisen) Ausbilden einer ersten mechanischen Verbindung zwischen dem Gehäuse 4 des Handstücks 2a und dem Griffstück 40 zunächst Fig. 12 das Griffstück 40 mit den Griffelementen 41 auf einen vorderen Gehäuseabschnitt 42 am (Handstück-)Gehäuse 4 aufgesteckt. Hierzu weist das Griffstück 40 sich nach hinten erstreckende, federnde Rastelemente 43 auf, die im gezeigten Beispiel mit Rasthaken gebildet und innenseitig umlaufend angeordnet sind. Die Rastelemente 43 verbinden sich formschlüssig mit einer umlaufenden Elementaufnahme 44 auf der Innenseite des Gehäuseabschnitts 42. In dieser Anordnung ist das Griffstück 40 (nach wie vor) von dem Gehäuse 4 und somit vom Handstück 2a (und somit der Antriebsvorrichtung 2) lösbar, indem das Griffstück 40 (wieder) abgenommen würde.

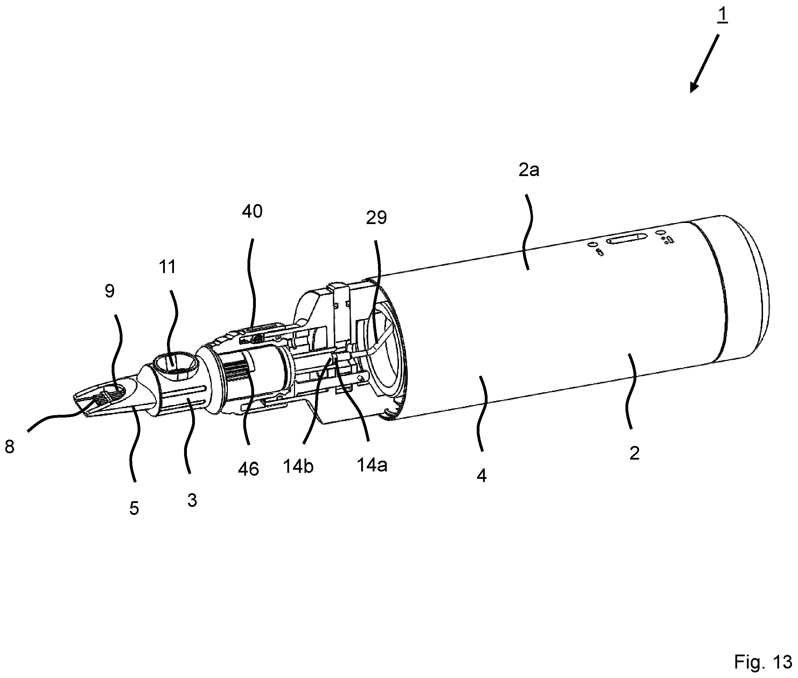

Sodann wird gemäß Fig. 13 das Hautstechmodul 3 in eine Aufnahme 45 des Griffstücks 40 eingesteckt und dort mittels einer Rasteinrichtung 46 verrastet, sodass eine drehfeste Verbindung zwischen Hautstechmodul 3 und Griffstück 40 ausgebildet wird. Hierdurch ist eine zweite mechanische Verbindung zwischen Hautstechmodul 3 und Griffstück 40 hergestellt. Das Ausbilden dieser zweiten mechanischen Verbindung führt dazu, dass nun das Griffstück 40 an dem vorderen Gehäuseabschnitt 42 des (Handstück-)Gehäuses 4 gesichert ist, da die Rastelemente 43 durch das eingesteckte Hautstechmodul 3 gesichert sind, derart, dass die Rastelemente 43 sich nicht mehr aus der Verbindung mit dem innenseitigen Elementaufnahme 44 lösen können. Hierdurch ist die erste mechanische Verbindung zwischen dem Gehäuse 4 des Handstücks 2a und dem Griffstück 40 dann gesichert ausgeführt.

Griffstück 40 und hiermit drehfest verbundenes Hautstechmodul 3 sind dann drehbar mit dem (Handstück-)Gehäuse 4 verbunden, derart, dass das Modulgehäuse 5 (zusammen mit dem Griffstück 40) in unterschiedliche Drehstellungen relativ zum Handstück 2a (oder Antriebsvorrichtung 2) verdreht oder verlagert werden kann. Im Betrieb kann der Nutzer so das Gehäuse 4, also das Handstück 2a, mit der Hand bzw. den Fingern fest greifen und je nach gewünschter Anpassung an unterschiedliche Anwendungsszenarien das Modulgehäuse 5 mit dem Griffstück 40 relativ hierzu drehen, so dass auch die Relativlage der vorderseitigen Öffnung 9 in Bezug auf das Handstück 2a geändert wird.

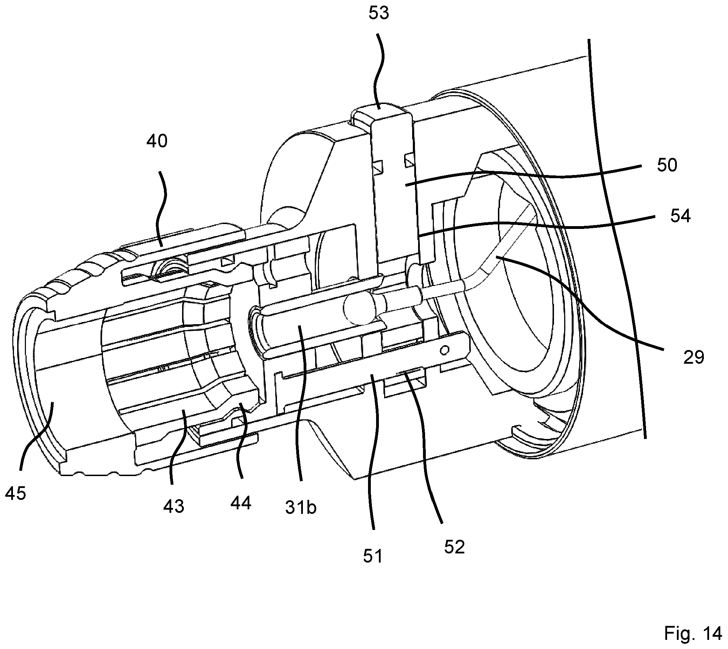

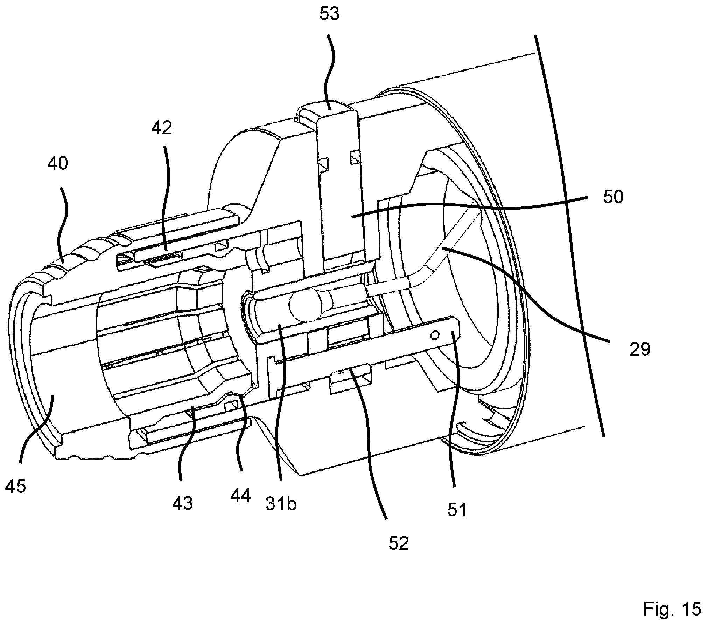

Fig. 14 und 15 zeigen eine Detaildarstellung mit aufgesetztem Griffstück 40 (vergleichbar Fig. 12). Es ist eine Einstelleinrichtung 50 zum Einstellen eines Nadelheraus- oder Nadelüberstands (NHS) gezeigt. Diese weist bei der gezeigten Ausführungsform einen in Längsrichtung verlaufenden Bolzen 51 auf, welcher hier beispielhaft T-förmig ausgeführt ist und auf seiner Oberfläche eine Verzahnung 52 aufweist, die mit einem Sicherungsbauteil 53 im Eingriff steht, welcher vorgespannt ist. Zum Verändern des Nadelherausstands kann das Sicherungsbauteil 53 in einer Bauteilaufnahme 54 manuell nach innen (in Fig. 14 und 15 nach unten) gedrückt werden (gegen eine Federkraft), so dass der Bolzen 51 freigegeben ist für ein Verschieben relativ zum Sicherungsbauteil 53 in Arbeitsrichtung. Fig. 14 und 15 zeigen unterschiedliche Stellungen des Bolzens 51 in Bezug auf das Sicherungsbauteil 53 und somit unterschiedliche Einstellungen für den Nadelherausstand. Dieser verändert sich, indem das Griffstück 40 zusammen mit dem hierin aufgenommen Hautstechmodul 3 in seiner Relativposition zur Verbindungsstelle 29 entlang der Arbeitsrichtung vor- oder zurück verschoben wird, wodurch sich (bei gleichbleibender Hublänge) der Heraus- oder Überstand der Nadelspitze 8 in Bezug auf die Modulgehäuseöffnung 9 verändert.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Antriebsvorrichtung (2) für ein Handgerät (1) zum lokalen Aufstechen einer Haut, mit
- einem Gehäuse (4), welches mit einem Hautstechmodul (3) verbindbar ist;
- einer Antriebseinrichtung (12), die in dem Gehäuse (4) angeordnet und eingerichtet ist, eine Antriebskraft mittels einer drehenden Antriebsbewegung einer Antriebswelle (17) bereitzustellen, deren Axialrichtung quer zu einer Arbeitsrichtung verläuft;
- einem Verbindungsbauteil (14), welches eingerichtet ist, getrieben von der Antriebskraft, entlang der Arbeitsrichtung eine Vor- und Zurückbewegung mit einer Hublänge auszuführen;
- einer Verbindungsstelle (14a), die an dem Verbindungsbauteil (14) gebildet und zum Verbinden mit einer Hautstecheinrichtung (7) des Hautstechmoduls (3) eingerichtet ist;
- einem Wandlungsmechanismus (13), welcher, die Antriebskraft übertragend, zwischen der Antriebswelle (17) und dem Verbindungsbauteil (14) in dem Gehäuse (4) angeordnet ist und eine Schwenkhebelanordnung aufweist, wobei bei dem Wandlungsmechanismus (13)
- die Schwenkhebelanordnung mit einem Schwenkhebel (15) gebildet ist, mit dem die drehende Antriebsbewegung der Antriebswelle (17) abgreifbar ist und der hierbei um einen Drehpunkt (16) schwenkt, der in Bezug auf die Antriebswelle (17) radial versetzt ist, und
- ein Pleuelbauteil (29) auf einer Seite mit einem Abgriff (22) an dem Schwenkhebel (15) und gegenüberliegend mit dem Verbindungsbauteil (14) verbunden ist; **gekennzeichnet durch**
- eine Hubeinstelleinrichtung (25), mit der zum Einstellen der Hublänge der Vor- und Zurückbewegung entlang der Arbeitsrichtung der Abgriff (22) an dem Schwenkhebel (15) verlagerbar ist.

2. Antriebsvorrichtung (2) nach Anspruch 1, **gekennzeichnet durch** eine Messeinrichtung, die der Hubeinstelleinrichtung (25) zugeordnet und eingerichtet ist, unterschiedliche Verlagerungsstellungen des Abgriffs (22) an dem Schwenkhebel (15) zu erfassen.

3. Antriebsvorrichtung (2) nach Anspruch 1 oder 2, **dadurch gekennzeichnet, dass** die Hubeinstelleinrichtung (25) mit einer manuellen Hubeinstelleinrichtung gebildet ist.

4. Antriebsvorrichtung (2) nach mindestens einem der vorangehenden Ansprüche, **dadurch gekennzeichnet, dass** die Hubeinstelleinrichtung (25) mit einer elektronischen Hubeinstelleinrichtung gebildet ist.

5. Antriebsvorrichtung (2) nach Anspruch 4, **dadurch gekennzeichnet, dass** die elektronischen Hubeinstelleinrichtung mit einer Steuereinrichtung verbunden ist, derart, dass von der Steuereinrichtung bereitstellbare Hubeinstellsignale an die elektronische Hubeinstelleinrichtung übertragbar sind.

6. Antriebsvorrichtung (2) nach mindestens einem der vorangehenden Ansprüche, **dadurch gekennzeichnet, dass** die Hubeinstelleinrichtung (25) eingerichtet ist, beim Verlagern des Abgriffs (22) an dem Schwenkhebel (15) einen Abstand des Abgriffs (22) zum Drehpunkt (16) des Schwenkhebels (15) zu ändern.

7. Antriebsvorrichtung (2) nach mindestens einem der vorangehenden Ansprüche, **gekennzeichnet durch** eine Abgriffeinrichtung zum Abgreifen der drehenden Antriebsbewegung der Antriebswelle (17), wobei die Abgriffeinrichtung ein Drehbauteil (18), welches mit der Antriebswelle (17) drehfest verbunden ist, und ein mit dem Drehbauteil (18) in Verbindung stehendes erstes Gleitbauteil (20) aufweist, welches in einer ersten Gleitführung (21) an dem Schwenkhebel (15) verlagerbar angeordnet ist.

8. Antriebsvorrichtung (2) nach mindestens einem der vorangehenden Ansprüche, **dadurch gekennzeichnet, dass** der Abgriff (22) an dem Schwenkhebel (15) mit einem zweiten Gleitbauteil (24) gebildet ist, welches in einer zweiten Gleitführung (23) an dem Schwenkhebel (15) verlagerbar angeordnet ist.

9. Antriebsvorrichtung (2) nach Anspruch 8, **dadurch gekennzeichnet, dass** eine Längsrichtung der ersten Gleitführung (21) und eine Längsrichtung der zweiten Gleitführung (23) einen spitzen Winkel einschließen.

10. Antriebsvorrichtung (2) nach Anspruch 8 oder 9, **dadurch gekennzeichnet, dass** das Pleuelbauteil (29) und das zweite Gleitbauteil (24) im Bereich des Abgriffs (22) relativ zueinander um eine Schwenkachse schwenkbar sind.

11. Antriebsvorrichtung (2) nach mindestens einem der vorangehenden Ansprüche, **dadurch gekennzeichnet, dass** die Hubeinstelleinrichtung (25) ein Einstellbauteil (28) aufweist, bei dem ein der Hubeinstelleinrichtung (25) zugeordnetes und mit dem Abgriff (22) verbundenes drittes Gleitbauteil (26) in einer dritten Gleitführung (27) an dem Einstellbauteil (28) verlagerbar angeordnet ist.

12. Antriebsvorrichtung (2) nach Ansprüchen 10 und 11, **dadurch gekennzeichnet, dass** das Pleuelbauteil (29) und das der Hubeinstelleinrichtung (25) zugeordnete dritte Gleitbauteil (26) im Bereich des Abgriffs (22) relativ zueinander um die Schwenkachse schwenkbar sind.

13. Antriebsvorrichtung (2) nach mindestens einem der vorangehenden Ansprüche, **dadurch gekennzeichnet, dass** die Arbeitsrichtung in Längsrichtung des Gehäuses (4) ausgebildet ist.

14. Handgerät (1) zum lokalen Aufstechen einer Haut, mit einer Antriebsvorrichtung (2) nach mindestens einem der vorangehenden Ansprüche und einem Hautstechmodul (3), welches mit dem Antriebsvorrichtung (2) verbunden ist.

## Claims

1. Drive device (2) for a hand-held device (1) for locally puncturing a skin, comprising
- a housing (4) which can be connected to a skin puncturing module (3);
- a drive device (12) which is arranged in the housing (4) and is configured to provide a drive force by means of a rotating drive movement of a drive shaft (17), the axial direction of which runs transversely with respect to a working direction;
- a connecting component (14) which is configured, driven by the drive force, to perform a forward and backward movement with a stroke length along the working direction;
- a connecting point (14a) which is formed on the connecting component (14) and is configured for connecting to a skin puncturing device (7) of the skin puncturing module (3);
- a conversion mechanism (13) which, in a manner which transmits the drive force, is arranged between the drive shaft (17) and the connecting component (14) in the housing (4) and has a pivoting lever arrangement, wherein, for the conversion mechanism (13),
- the pivoting lever arrangement is formed with a pivoting lever (15) with which the rotating drive movement of the drive shaft (17) can be tapped and which in the process pivots about a pivot point (16) which is offset radially with respect to the drive shaft (17), and
- a connecting rod component (29) is connected, on one side, to a tap (22) on the pivoting lever (15) and, opposite, to the connecting component (14); **characterized by**
- a stroke setting device (25) with which the tap (22) on the pivoting lever (15) can be displaced in order to set the stroke length of the forward and backward movement along the working direction.

2. Drive device (2) according to Claim 1, **characterized by** a measuring device which is assigned to the stroke setting device (25) and is configured to detect different displacement positions of the tap (22) on the pivoting lever (15).

3. Drive device (2) according to Claim 1 or 2, **characterized in that** the stroke setting device (25) is formed with a manual stroke setting device.

4. Drive device (2) according to at least one of the preceding claims, **characterized in that** the stroke setting device (25) is formed with an electronic stroke setting device.

5. Drive device (2) according to Claim 4, **characterized in that** the electronic stroke setting device is connected to a control device in such a way that stroke setting signals which can be provided by the control device can be transmitted to the electronic stroke setting device.

6. Drive device (2) according to at least one of the preceding claims, **characterized in that** the stroke setting device (25) is configured to change a distance of the tap (22) from the pivot point (16) of the pivoting lever (15) when the tap (22) on the pivoting lever (15) is displaced.

7. Drive device (2) according to at least one of the preceding claims, **characterized by** a tapping device for tapping the rotating drive movement of the drive shaft (17), wherein the tapping device has a rotating component (18) which is connected to the drive shaft (17) in a rotationally fixed manner, and a first sliding component (20) which is connected to the rotating component (18) and is arranged in a displaceable manner in a first sliding guide (21) on the pivoting lever (15).

8. Drive device (2) according to at least one of the preceding claims, **characterized in that** the tap (22) on the pivoting lever (15) is formed with a second sliding component (24) which is arranged in a displaceable manner in a second sliding guide (23) on the pivoting lever (15).

9. Drive device (2) according to Claim 8, **characterized in that** a longitudinal direction of the first sliding guide (21) and a longitudinal direction of the second sliding guide (23) enclose an acute angle.

10. Drive device (2) according to Claim 8 or 9, **characterized in that** the connecting rod component (29) and the second sliding component (24) are pivotable relative to one another about a pivot axis in the region of the tap (22).

11. Drive device (2) according to at least one of the preceding claims, **characterized in that** the stroke setting device (25) has a setting component (28) in which a third sliding component (26) which is assigned to the stroke setting device (25) and is connected to the tap (22) is arranged in a displaceable manner in a third sliding guide (27) on the setting component (28).

12. Drive device (2) according to Claims 10 and 11, **characterized in that** the connecting rod component (29) and the third sliding component (26) which is assigned to the stroke setting device (25) are pivotable relative to one another about the pivot axis in the region of the tap (22).

13. Drive device (2) according to at least one of the preceding claims, **characterized in that** the working direction is formed in the longitudinal direction of the housing (4).

14. Hand-held device (1) for locally puncturing a skin, comprising a drive device (2) according to at least one of the preceding claims and a skin puncturing module (3) which is connected to the drive device (2).

## Revendications

1. Dispositif d'entraînement (2) pour appareil à main (1) pour le perçage local d'une peau, avec
- un boîtier (4) qui peut être relié à un module de piqûre cutanée (3) ;
- un dispositif d'entraînement (12), disposé et conçu dans le boîtier (4), pour fournir une force motrice par un mouvement rotatif d'un arbre d'entraînement (17) dont la direction axiale est transversale à un sens de travail ;
- un composant de liaison (14), qui est conçu pour exécuter, en étant entraîné par la force motrice, un mouvement d'avant en arrière le long du sens de travail sur une longueur de course ;
- un point de liaison (14a) qui est formé sur le composant de liaison (14) et conçu pour être connecté à un dispositif de piqûre cutanée (7) du module de piqûre cutanée (3) ;
- un mécanisme de conversion (13) qui est disposé dans le boîtier (4), en transmettant la force d'entraînement, entre l'arbre d'entraînement (17) et le composant de connexion (14), et doté d'un dispositif de levier pivotant, sachant que, pour le mécanisme de conversion (13),
- l'agencement de levier pivotant est formé par un levier pivotant (15) permettant de saisir le mouvement d'entraînement rotatif de l'arbre d'entraînement (17) et pivotant d'un point de pivotement (16) qui est décalé radialement par rapport à l'arbre d'entraînement (17), et
- une bielle (29) coudée est reliée d'un côté par une poignée (22) au levier pivotant (15) et opposée à la pièce de liaison (14) ;
**caractérisé par**
- un dispositif de levage (25) permettant de déplacer la poignée (22) par le levier pivotant (15) pour régler la longueur de course du mouvement d'avant en arrière le long du sens de travail.

2. Dispositif d'entraînement (2) selon la revendication 1, **caractérisé par** un dispositif de mesure qui est affecté au dispositif de levage (25) et conçu pour détecter les différentes positions de déplacement de la poignée (22) sur le levier pivotant (15).

3. Dispositif d'entraînement (2) selon la revendication 1 ou 2, **caractérisé en ce que** le dispositif de levage (25) est constitué d'un dispositif de levage manuel.

4. Dispositif d'entraînement (2) selon au moins l'une des revendications précédentes, **caractérisé en ce que** le dispositif de levage (25) est constitué d'un dispositif de levage électronique.

5. Dispositif d'entraînement (2) selon la revendication 4, **caractérisé en ce que** le dispositif de levage électronique est connecté à un dispositif de commande, de telle sorte que les signaux de levage fournis par le dispositif de commande peuvent être transmis au dispositif de levage électronique.

6. Dispositif d'entraînement (2) selon au moins une des revendications précédentes, **caractérisé en ce que** le dispositif de levage (25) est conçu, lors du déplacement de la poignée (22) sur le levier pivotant (15), pour modifier une distance de la poignée (22) au point de pivotement (16) du levier pivotant (15) par rapport à l'autre.

7. Dispositif d'entraînement (2) selon au moins une des revendications précédentes, **caractérisé par** un dispositif de préhension permettant de capter le mouvement d'entraînement rotatif de l'arbre d'entraînement (17), le dispositif de préhension étant muni d'un composant rotatif (18) relié à l'arbre d'entraînement (17) et un premier élément de glissement (20) relié au composant rotatif (18), qui peut être déplacé dans un premier guide coulissant (21) sur le levier pivotant (15).

8. Dispositif d'entraînement (2) selon au moins l'une des revendications précédentes, **caractérisé en ce que** la poignée (22) sur le levier pivotant (15) est formée d'un deuxième élément de glissement (24) qui est disposé dans un second guide de glissement (23) sur le levier pivotant (15).

9. Dispositif d'entraînement (2) selon la revendication 8, **caractérisé en ce qu'**un sens longitudinal du premier guide de glissement (21) et un sens longitudinal du second guide de glissement (23) comprennent un angle aigu.

10. Dispositif d'entraînement (2) selon la revendication 8 ou 9, **caractérisé en ce que** la bielle (29) et le deuxième élément de glissement (24) sont pivotants l'un par rapport à l'autre dans la zone de la poignée (22) autour d'un axe de pivotement.

11. Dispositif d'entraînement (2) selon au moins une des revendications précédentes, **caractérisé en ce que** le dispositif de levage (25) comporte un composant de réglage (28) dans lequel un troisième élément de glissement (26) associé au dispositif de levage (25) et relié à la poignée (22) est positionné de manière déplaçable dans un troisième guide de glissement (27) sur le composant de réglage (28).

12. Dispositif d'entraînement (2) selon les revendications 10 et 11, **caractérisé en ce que** la bielle (29) et le troisième élément de glissement (26) associé au dispositif de levage (25) peuvent pivoter l'un par rapport à l'autre dans la zone de la poignée (22) autour de l'axe de pivotement.

13. Dispositif d'entraînement (2) selon au moins l'une des revendications précédentes, **caractérisé en ce que** le sens de travail est établi dans le sens longitudinal du boîtier (4).

14. Appareil à main (1) pour le perçage local d'une peau, avec un dispositif d'entraînement (2) selon au moins l'une des revendications précédentes et un module de piqûre cutanée (3) relié au dispositif d'entraînement (2).
